# EUROPEAN PATENT SPECIFICATION

(11) **EP 1 406 614 B1**
(45) Date of publication and mention of the grant of the patent: **07.06.2006**
(21) Application number: 02761099.7
(22) Date of filing: 15.07.2002
(51) Int. Cl.: A61K 31/403, C07D 209/88

(54) **CARVEDILOL POLYMORPH**
CARVEDILOLPOLYMORPH
POLYMORPHE DE CARVEDILOL

(30) Priority: 13.07.2001 US 305593 P; 22.08.2001 US 314150 P
(43) Date of publication of application: 14.04.2004
(73) Proprietor: SMITHKLINE BEECHAM CORPORATION, Philadelphia, Pennsylvania 19101 (US)
(72) Inventor: CHEN, Wei, Collegeville, PA 19426 (US); GALLOP, Marc, Norristown, PA 19403 (US); OH, Choon, K., Collegeville, PA 19426 (US)
(74) Representative: Shore, Andrew David
(86) International application number: PCT/US2002/022374
(87) International publication number: WO 2003/005970

(56) References cited:
- WO-A1-99/05105
- US-A- 4 503 067

## Description

### FIELD OF THE INVENTION

This invention relates to a novel crystalline form of Carvedilol.

### BACKGROUND OF THE INVENTION

The capacity to occur in different crystal structures is known as polymorphism and is known to occur in many organic compounds. These different crystalline forms are known as "polymorphic modifications" or "polymorphs" and are realized in their crystalline state. While polymorphic modifications have the same chemical composition, they differ in packing, geometrical arrangement, and other descriptive properties of the crystalline solid state. As such, these modifications may have different solid-state physical properties such as shape, color density, hardness, deformability, stability, and dissolution properties, etc. Polymorphism of an organic drug molecule and its consequences will be appreciated by the skilled artisan.

Carvedilol is 1-(carbazol-4-yloxy-3-[[2-(o-methoxyphenoxy)ethyl]amino]-2-propanol, and has the following structural formula:

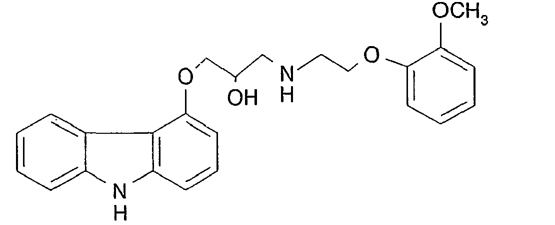

Carvedilol is the subject of U.S. Patent No. 4,503,067 (the '067 patent), issued March 5, 1985, and of WO 99/05105, published on 04. Feb. 1999.

Carvedilol is useful in the treatment of hypertension, congestive heart failure and angina.

According to the instant invention, it has been found that carvedilol exists in a novel crystalline form.

### SUMMARY OF THE INVENTION

This invention relates to a novel crystalline form of carvedilol which is useful in the treatment of hypertension, congestive heart failure and angina.

### BRIEF DESCRIPTION OF THE FIGURES

Figure 1 is a MDSC thermogram of carvedilol spray-dried powder (Batch N00189).
Figure 2 is a MDSC thermogram of carvedilol spray-dried powder (Batch N00190).
Figure 3 is a MDSC thermogram of carvedilol spray-dried powder (Batch N00191).
Figure 4 are XRPD patterns for batches of carvedilol spray-dried powder and the reference standard of carvedilol drug substance (Form II).

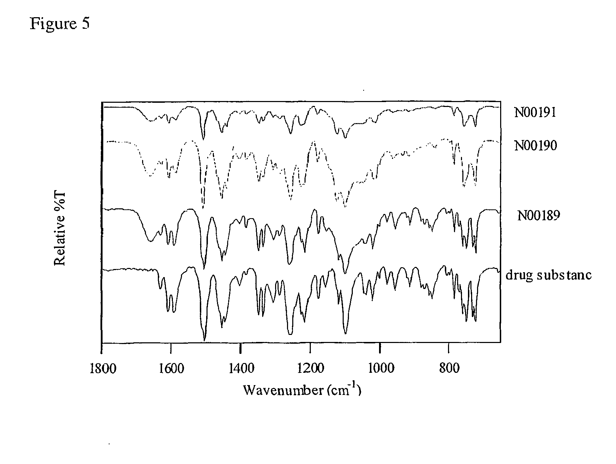

Figure 5 are FT-IR for batches of carvedilol spray-dried powder and the reference standard of carvedilol drug substance (Form II).
Figure 6 is a MDSC thermogram of carvedilol drug substance (Form II).
Figure 7 is a MDSC thermogram of carvedilol Form III.
Figure 8a is the FT-IR spectra (400-1200 cm⁻¹ region) of carvedilol Form II and Form III.
Figure 8b is the FT-IR spectra (2500-4000 cm⁻¹ region) of carvedilol Form II and Form III.
Figure 9 are the XRPD patterns for carvedilol Form III (Lot 46233-138), Form II and carvedilol spray-dried powder (Lot N00191).
Figure 10 is the ¹³C NQS-edited CP-TOSS spectra for carvedilol Form II and Form III.
Figure 11 is a comparison of ¹⁵N CP-MAS spectra for carvedilol Form II and Form III.

### DETAILED DESCRIPTION OF THE INVENTION

It has now been discovered that Carvedilol can exist as a novel crystalline form (a novel polymorphic form) which differs from previously known forms in its stability, physical properties and spectral data. This novel form is described in this application and is herein referred to as Form III.

This invention also relates to a pharmaceutical composition comprising an effective amount of Form III with any of the characteristics noted herein, and a pharmaceutically acceptable carrier or diluent thereof.

This invention further relates to the use for treatment of hypertension, congestive heart failure and angina in a mammal in need thereof, which method comprises administering to said mammal an effective amount of Form III with any of the characteristics noted herein.

This invention results from a determination that certain batches of Carvedilol showed differences in their IR spectra, DSC and X-ray powder diffraction pattern. The novel crystalline form of carvedilol of the instant invention may be produced during the wet-bead milling process of Carvedilol Form II, the current commercial form of Carvedilol. The manufacturing process used to prepare the novel polymorphic form of carvedilol included the following steps: 1) preparing Carvedilol suspension which contained 30% drug and 6% Pluronic® F127, 2) wet-bead milling of the Carvedilol suspension, 3) mixing the milled Carvedilol suspension with HEC/PVP solution, and 4) spray drying of the final suspension. The carvediol spray-dried powder produced during this manufacturing process contained 66.7% drug, 13.3% Pluronic® F127, 6.7% Poly(Vinyl Pymolidone) (PVP) K30, 13.3% Hydroxyethylcellulose (HEC).

Carvedilol Form III may also be prepared by stirring Carvedilol Form II in water at about 60° C. This novel crystalline form melts at about 92-95° C, while Form II melts at about 114-115° C.

### Experimental Details:

The procedure to prepare the new crystalline form is summarized below:
1. Add water into a glass beaker (or flask), and stir vigorously using a magnetic stir bar. Slowly add Carvedilol Form II powder into water. The Carvedilol to water ratio used has been between 1:200 and 1:40 gram/ml.
2. Heat the suspension to 60-65 °C. Keep stirring at this temperature with the container being covered by aluminum foil.
3. Monitor the conversion process by analyzing the suspension using DSC as described below. Usually it takes 2 to 3 days for the conversion to complete.
4. Separate the solids from liquid by filtration when the conversion is complete. Wash the solids with water a couple of times. Subsequently dry the obtained solids in a desiccator under vacuum, or at 60 °C under vacuum.

### Methods

### Differential Scanning Calorimetry (DSC):

DSC measurements were performed with a MDSC 2920 (TA Instruments, Inc.). About 5 mg of the powder was placed in an open aluminum pan. The sample was scanned at 10 °C/min from 0 °C to 160 °C. The suspension sample was placed in a plastic weighing dish and excess liquid was removed manually by paper tissues. The solids were then dried in a fume hood before DSC measurements.

### Fourier Transform Infrared Spectroscopy (FT-IR):

Approximately 2 mg of sample was diluted with 300 mg of dried potassium bromide (KBr). The mixture was ground with a mortar and pestle, then transferred to a die that is placed under high pressure for 3 minutes. The instrument was a PerkinElmer Spectrum GX FTIR instrument. Ten scans were collected at 4 cm⁻¹ resolution.

### X-Ray Powder Diffraction (XRPD):

XRPD patterns were collected using a Bruker D8 Advance X-ray Diffractometer. Approximately 60 mg of sample was gently flattened on a silicon sample holder and scanned from 2 to 35 degrees two-theta, at 0.02 degrees two-theta per step and a step time of 2.5 seconds. The suspension samples were simply placed on the sample holder. The sample was rotated at 25 rpm.

### Solid State Nuclear Magnetic Resonance (Solid-State NMR):

Approximately 500 mg of each sample was packed into 7-mm magic-angle spinning rotors and spun at 5 kHz. The NQS (*n*on-*q*uaternary *s*uppression) edited CP-MAS pulse sequence (*c*ross-*p*olarization with *m*agic *a*ngle *s*pinning) was used to measure the ¹⁵N spectrum of the samples. The ¹³C spectrum of each sample was recorded using a CP-TOSS pulse sequence (*c*ross *p*olarization with *to*tal suppression of sidebands). A Brucker AMX/ARX-360 spectrometer was used for this work.

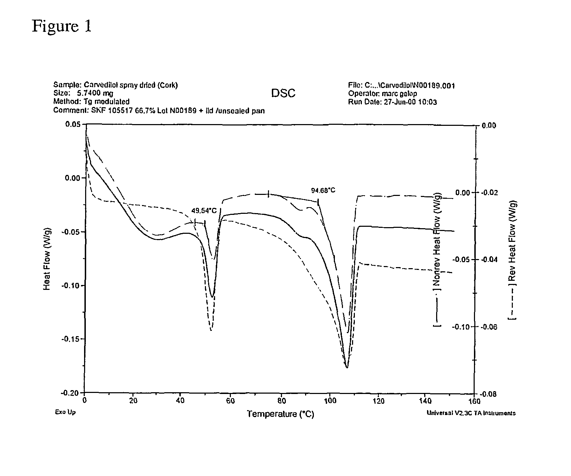

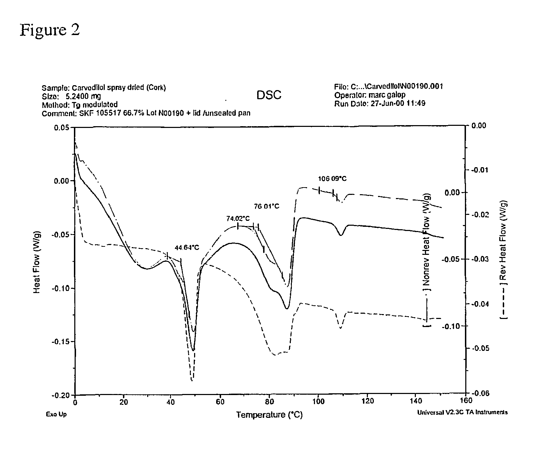

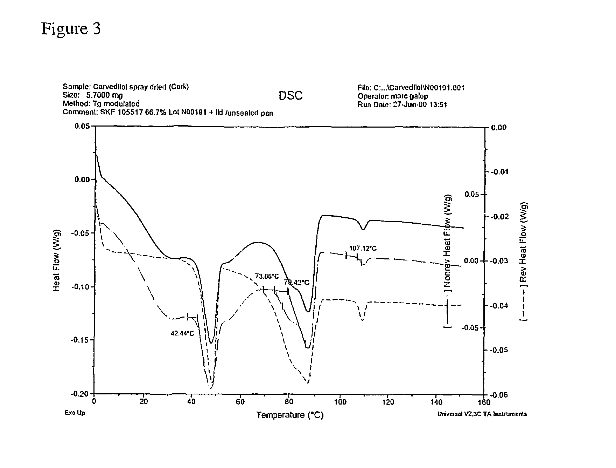

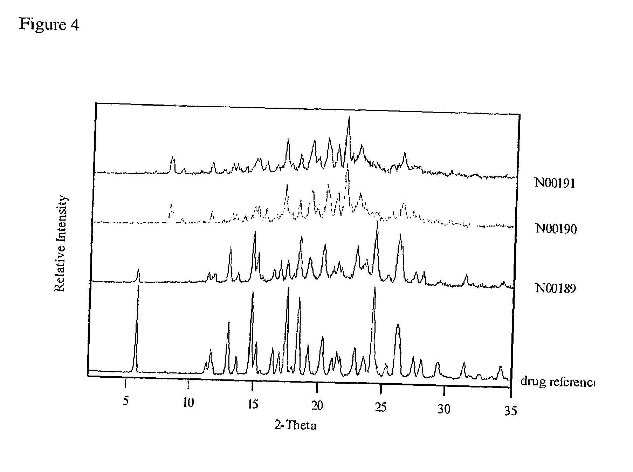

Figures 1 to 3 show the MDSC thermograms of three batches (N00189, N00190 and N00191) of Carvedilol spray-dried powder. Figure 4 is the XRPD patterns for the batches of Carvedilol spray-dried powder together with the reference standard of Carvedilol drug substance.

There were two major endothermic peaks on the thermogram of N00189, near 50 °C and 90 °C, corresponding to the melting of Pluronic F127 and the drug, respectively. It should be noted that the melting onset temperature of the drug was much lower than the pure drug (113-115 °C for Carvedilol Form II). This was due to the interactions between the drug particles and Pluronic F127 adsorbed on the surface of the drug particles. Both XRPD pattern and FT-IR spectrum for N00189 demonstrated that Carvedilol existed as Form II in the spray-dried powder, same as the reference standard of the drug substance.

Batches N00190 and N00191 spray-dried powder were different from batch N00189. Indeed, a new endothermic peak appeared near 75 °C on the thermograms of these two batches. This endothermic peak corresponded to the melting of a distinct and new crystalline form. This new crystalline form exhibited very different XRPD patterns and FT-IR spectra from the Form II drug substance.

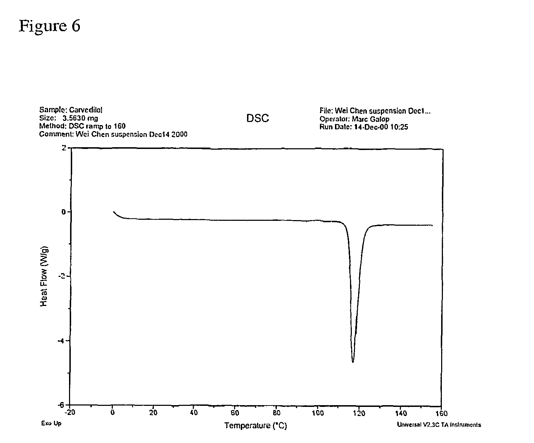

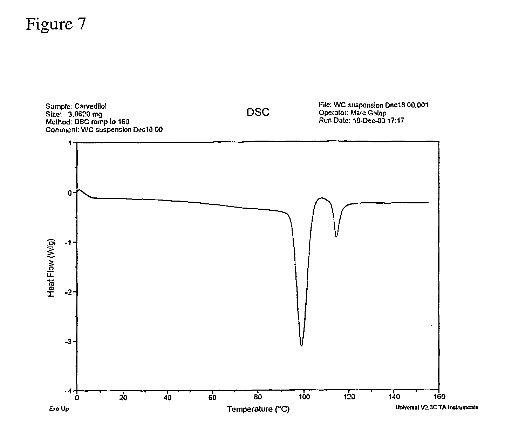

Figures 6 and 7 show the typical thermograms of Carvedilol Form II and Form III, respectively.

As shown in Figure 7, the thermogram of the new material exhibited a major endotherm (melting event) near 92 °C, immediately followed by a small exotherm (recrystallization), and a small endotherm near 110 °C. One possible explanation for this behavior is that, after the new crystalline form melted, a small amount of the melt recrystallized into Carvedilol Form II and then gave the second melting peak.

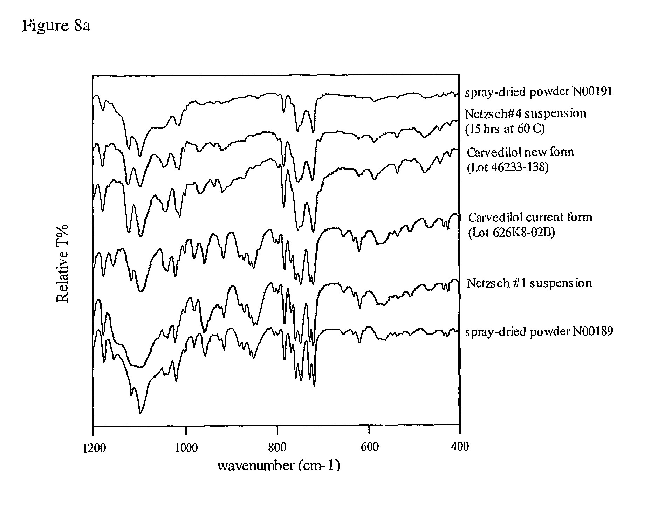

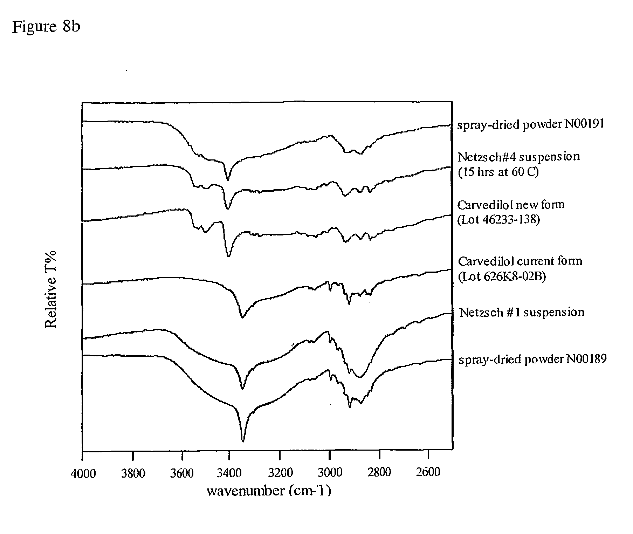

Figures 8a and 8b show a comparison of FT-IR spectra for the new form and the currently used form (Form II) in drug substance, milled suspensions and spray-dried powders. It can be seen that the drug exists as Form II in Netzsch#1 milled suspension and spray-dried powder N00189. Most importantly, Form III (Lot 46233-138) exhibited the same FT-IR spectral features as the new form discovered in spray-dried powder N00191 and heat-treated Netzsch#4 suspension. Therefore, the new form material prepared by stirring Carvedilol Form II suspension is the same crystalline form as the one obtained in the two batches of Carvedilol spray-dried powder.

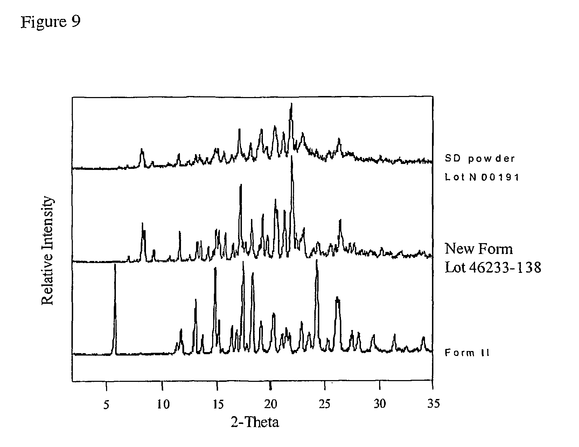

The XRPD pattern for the new form material is displayed in Figure 9 together with the currently used form of Carvedilol (Form II) and Carvedilol spray-dried powder Lot N00191. Again, the diffraction pattern from the new form material matched that from the spray-dried powder Lot N00191, indicating the same crystalline structure. Carvedilol Form III has an X-ray powder diffraction pattern which comprises characteristic peaks at about 8.4, 17.4 and 22.0 degrees two-theta.

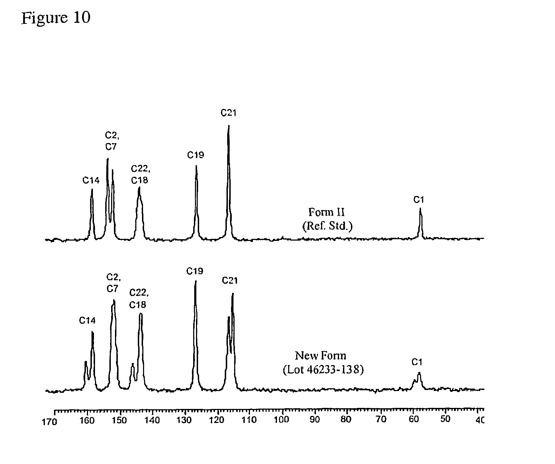

The ¹³C NQS-edited CP-TOSS spectra for the Carvedilol Form III and Form II are illustrated in Figure 10, where only quaternary and methyl resonances are present. The assignments of each peak were made, in reference to the structure of Carvedilol (Scheme 1).

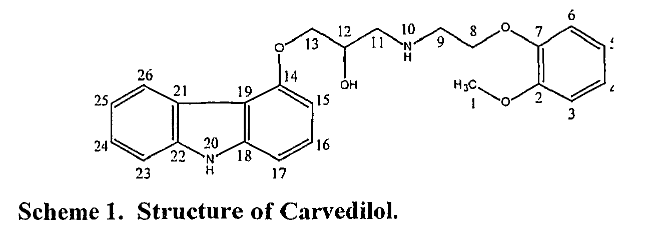

From its NMR spectrum, it appears that Form II sample contains one molecule per crystalline unit cell. However, the typical splitting of peaks observed in compounds with more than one molecule per unit cell is observed for the Carvedilol new form. The splitting effect could also arise from a mixture of two or more phases; however, since the spectra do not coincide, these phases must also be new.

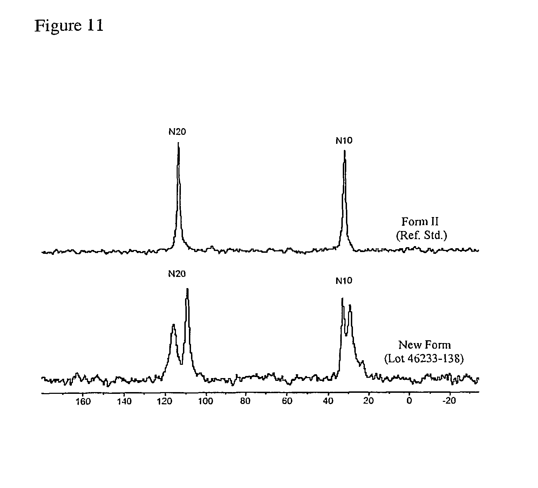

The ¹⁵N CP-MAS spectra of the two forms are shown in Figure 11. Again, Form II appears to contain a single molecule per unit cell, while the Form III contains multiple resonances for each type of nitrogen, consistent with either multiple molecules per unit cell or multiple new phases.

The above data demonstrates that a new crystalline form of Carvedilol (Form III) is prepared from Carvedilol Form II.

## Claims

1. A compound which is carvedilol Form III, obtainable by the following method:
a. add water into a glass beaker (or flask), and stir vigorously using a magnetic stir bar, slowly add Carvedilol Form II powder into water, the Carvedilol to water ratio used being between 1:200 and 1:40 gram/ml.
b. heat the suspension to 60-65 °C, keep stirring at this temperature with the container being covered by aluminum foil.
c. monitor the conversion process by analyzing the suspension using DSC, between 2 to 3 days for the conversion to complete.
d. separate the solids from liquid by filtration when the conversion is complete, wash the solids with water a couple of times, subsequently dry the obtained solids in a desiccator under vacuum, or at 60 °C under vacuum.

2. The compound according to claim 1 having an X-ray powder diffraction pattern which comprises characteristic peaks as expressed in Figure 9, Lot 46233-138.

3. The compound according to claim 1 having an X-ray powder diffraction pattern which comprises characteristic peaks at about 8.4, 17.4 and 22.0 degrees two-theta.

4. The compound according to claim 1 having an infrared spectrum which comprises characteristic absorption bands expressed in reciprocal centimeters as described in Figures 8a and 8b, Lot 46233-138.

5. The compound according to claim 1 having a DSC enothermic peak at about 92 °C.

6. The compound according to claim 1 having a melting point at about 92-95 °C.

7. A pharmaceutical composition comprising the compound according to any one of claims 1 to 6, and a pharmaceutically acceptable carrier or diluent.

8. The use of a compound according to any one of claims 1 to 6 in the manufacture of a medicament for use in the treatment of hypertension, angina or congestive heart failure.

## Patentansprüche

1. Verbindung, die Carvedilol Form III ist, erhältlich durch das folgende Verfahren:
(a) Zugeben von Wasser in ein Becherglas (oder einen Kolben) und kräftiges Rühren unter Verwendung eines Magnetrührers, langsames Zugeben von Pulver aus Carvedilol Form II in das Wasser, wobei das verwendete Verhältnis von Carvedilol zu Wasser zwischen 1:200 und 1:40 g/ml ist,
(b) Erwärmen der Suspension auf 60 bis 65°C, Fortsetzen des Rührens bei dieser Temperatur, wobei der Behälter mit Aluminiumfolie bedeckt ist,
(c) Überwachen des Umwandlungsprozesses durch Analysieren der Suspension unter Verwendung von DSC für 2 bis 3 Tage auf Vollständigkeit der Umwandlung,
(d) Abtrennen der Feststoffe von der Flüssigkeit durch Filtration, wenn die Umwandlung vollständig ist, mehrmaliges Waschen der Feststoffe mit Wasser, anschliessendes Trocknen der erhaltenen Feststoffe in einem Exsikkator unter Vakuum oder bei 60°C unter Vakuum.

2. Verbindung gemäss Anspruch 1 mit einem Röntgen-Pulverbeugungsmuster, das charakteristische Peaks wie in Fig. 9, Charge 46233-138 ausgedrückt umfasst.

3. Verbindung gemäss Anspruch 1 mit einem Röntgen-Pulverbeugungsmuster, das charakteristische Peaks bei ca. 2θ = 8,4°, 17,4° und 22,0° umfasst.

4. Verbindung gemäss Anspruch 1 mit einem Infrarotspektrum, das charakteristische Absorptionsbanden, ausgedrückt in cm⁻¹, wie in Fig. 8a und 8b, Charge 46233-138 umfasst.

5. Verbindung gemäss Anspruch 1 mit einem endothermen DSC-Peak bei ca. 92°C.

6. Verbindung gemäss Anspruch 1 mit einem Schmelzpunkt bei ca. 92 bis 95°C.

7. Pharmazeutische Zusammensetzung, die die Verbindung gemäss einem der Ansprüche 1 bis 6 und einen pharmazeutisch annehmbaren Träger oder Verdünnungsstoff umfasst.

8. Verwendung einer Verbindung gemäss einem der Ansprüche 1 bis 6 in der Herstellung eines Medikaments zur Verwendung in der Behandlung von Hypertonie, Angina oder Stauungsherzinsuffizienz.

## Revendications

1. Composé qui est le carvédilol Forme III, pouvant être obtenu par le procédé suivant :
- (a) introduire de l'eau dans un bécher (ou un flacon) en verre et agiter vigoureusement à l'aide d'un barreau magnétique, ajouter lentement du carvédilol Forme II en poudre dans l'eau, le rapport utilisé carvédilol/eau étant compris entre 1:200 et 1:50 g/ml ;
- (b) chauffer la suspension à 60-65°C et maintenir l'agitation à cette température, le récipient étant couvert par une feuille d'aluminium;
- (c) suivre le processus de conversion en analysant la suspension par Calorimétrie à Balayage Différentiel (CBD) pendant 2 à 3 jours jusqu'à conversion complète ;
- (d) lorsque la conversion est complète, séparer les solides du liquide par filtration, laver les solides à l'eau à deux reprises, et ensuite sécher les solides obtenus dans un dessiccateur sous vide ou à 60°C sous vide.

2. Composé selon la revendication 1, ayant un profil de diffraction de poudre aux rayons X comprenant des pics caractéristiques tels que ceux représentés en Figure 9, Lot 46233-138.

3. Composé selon la revendication 1, ayant un profil de diffraction de poudre aux rayons X comprenant des pics caractéristiques à environ 8,4, 17,4 et 22,0 degrés deux-théta.

4. Composé selon la revendication 1, ayant un spectre infrarouge qui comprend des bandes d'absorption exprimées en inverse de centimètre, telles que celles représentées en Figures 8a et 8b, Lot 46233-138.

5. Composé selon la revendication 1, ayant en CBD un pic endotherme à environ 92°C.

6. Composé selon la revendication 1, ayant un point de fusion d'environ 92-95°C.

7. Composition pharmaceutique comprenant le composé selon l'une quelconque des revendications 1 à 6, et un véhicule ou diluant pharmaceutiquement acceptable.

8. Utilisation d'un composé selon l'une quelconque des revendications 1 à 6, dans la fabrication d'un médicament destiné à être utilisé dans le traitement de l'hypertension, de l'angine de poitrine ou de la défaillance congestive du coeur.
